# EUROPÄISCHE PATENTSCHRIFT

(11) **EP 1 188 750 B1**
(45) Veröffentlichungstag und Bekanntmachung des Hinweises auf die Patenterteilung: **15.10.2003**
(21) Anmeldenummer: 01130001.9
(22) Anmeldetag: 15.12.1999
(51) Int. Cl.: C07D 239/48

(54) **Verfahren zur Herstellung von 2,5-Diamino-4,6-dihalogenpyrimidinen**
Process for the preparation of 2,5-diamino-4,6-dihalogenopyrimidinen
Procédé de préparation de 2,5-diamino-4,6-dihalogénopyrimidines

(30) Priorität: 21.12.1998 EP 98124188; 18.01.1999 EP 99100788; 12.04.1999 EP 99107161
(43) Veröffentlichungstag der Anmeldung: 20.03.2002
(62) Teilanmeldung aus: 99125042.4
(73) Patentinhaber: Lonza AG, 4052 Basel (CH)
(72) Erfinder: Saikali, Elie, Dr., 19490 Worcester, PA (US); Brieden, Walter, Dr., 3902 Brig-Glis (CH)

(56) Entgegenhaltungen:
- EP-A- 0 684 236
- WO-A-91/01310
- US-A- 4 965 270

## Beschreibung

Die Erfindung betrifft ein neues Verfahren zur Herstellung eines 2,5-Diamino-4,6-dihalogenpyrimidins der allgemeinen Formel 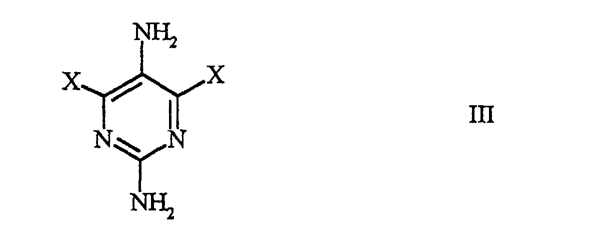 worin X ein Halogenatom bedeutet.

Wie in der parallelen EP-A-1 013 647 beschrieben dienen 2,5-Diamino-4,6-dihalogenpyrimidine der allgemeinen Formel m als Ausgangsmaterial zur Herstellung von N-(Amino-4,6-dihalogenpyrimidin)formamiden der Formeln 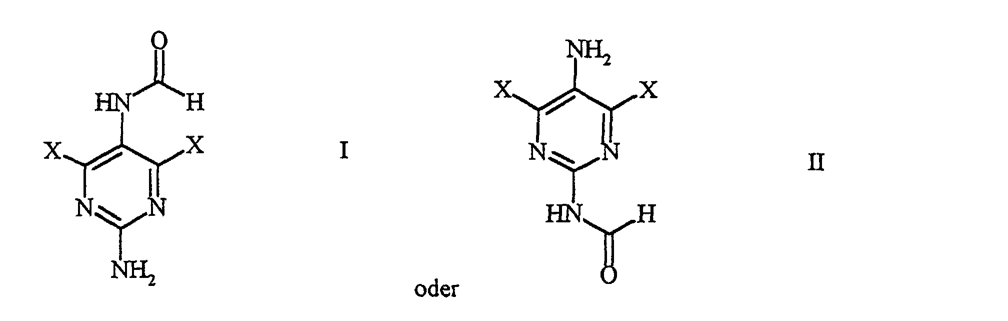 die wiederum wichtige Zwischenprodukte zur Herstellung von antiviralen Nukleotidderivaten sind (EP-A 0 684 236).

Bisher sind mehrere Verfahren zur Herstellung von 2,5-Diamino-4,6-dihalogenpyrimidinen wie 2,5-Diamino-4,6-dichlorpyrimidin bekannt. Beispielsweise beschreibt die WO 91/01310 ein Verfahren zur Herstellung von 2,5-Diamino-4,6-dichlorpyrimidin ausgehend von 2,5-Diamino-4,6-dihydroxypyrimidin in Gegenwart von Phosphoroxychlorid und einem quartären Ammoniumhalogenid oder einem schwach basischen tertiären Amin bzw. dessen Salz. Dabei dient das Phosphoroxychlorid als Lösungsmittel. Dieses Verfahren hat den Nachteil, dass es im grosstechnischen Masstab nicht reproduzierbar ist und das gewünschte Endprodukt nur in geringer Ausbeute erhalten wird.

Aufgabe der vorliegenden Erfindung war es, ein einfacheres Verfahren zur Herstellung von 2,5-Diamino-4,6-dihalogenpyrimidinen bereit zu stellen, bei dem das gewünschte Produkt in guter Ausbeute erhalten wird.

Diese Aufgabe wurde durch das Verfahren nach Anspruch 1 gelöst, wonach 2,5-Diamino-4,6-dihalogenpyrimidine der allgemeinen Formel III, in guter Ausbeute erhalten werden, wenn man 2,5-Diamino-4,6-dihydroxypyrimidin der Formel IV 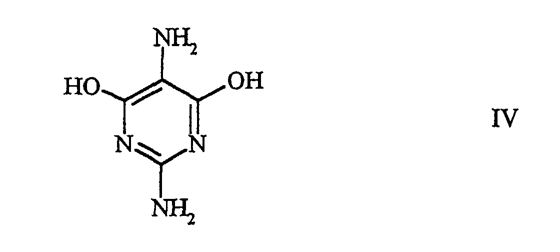 oder eines seiner Salze in Gegenwart eines Phosphoroxyhalogenids und eines quartären Ammoniumhalogenids mit einem halogenierten Alkan als Lösungsmittel umsetzt.

Das 2,5-Diamino-4,6-dihydroxypyrimidin ist eine käufliche Verbindung. Geeignet für das Verfahren sind auch die Salze von 2,5-Diamino-4,6-dihydroxypyrimidin, beispielsweise die Hydrohalogenidsalze wie die Hydrochloridsalze bzw. Hydrobromidsalze.

Als Phosphoroxyhalogenid wird zweckmässig Phosphoroxychlorid oder Phosophoroxybromid eingesetzt.

Geeignete quartäre Ammoniumhalogenide sind beispielsweise Tetraalkylammoniumhalogenide, worin die Alkylreste gleich oder verschieden sein können und vorzugsweise C₁₋₆-Alkyl, insbesondere Methyl, Ethyl, Propyl oder Butyl bedeuten. Zu nennen sind hier beispielsweise Tetramethylammoniumhalogenide oder Tetraethylammoniumhalogenide. Zweckmäßig werden als Halogenide der quarternären Ammoniumverbindungen die Chloride oder Bromide eingesetzt.

Üblicherweise wird das quartäre Ammoniumhalogenid im Überschuss, bezogen auf das 2,5-Diamino-4,6-dihydroxypyrimidin, eingesetzt; vorzugsweise werden 1 bis 10 mol quartäres Ammoniumhalogenid pro 1 mol 2,5-Diamino-4,6-dihydroxypyrimdin eingesetzt.

Zweckmässig wird die Umsetzung bei einer Temperatur von 20°C bis zur Rückflusstemperatur des entsprechenden Lösungsmittels, vorzugsweise von 100 bis 120°C, durchgeführt.

Als halogeniertes Alkan kann halogeniertes Propan wie 1,2,3-Trichlorpropan eingesetzt werden.

Wie in der EP-A-1 013 647 beschrieben, können die N-(Amino-4,6-dihalogenpyrimidin)formamide der Formeln I und II direkt, d. h. ohne Zwischenstufen, in hervorragender Ausbeute erhalten werden, indem die 2,5-Diamino-4,6-dihalogenpyrimidine der allgemeinen Formel III oder eines ihrer Salze mit Ameisensäure umsetzt.

Halogenatome X in der Verbindung der allgemeinen Formel III sind beispielsweise Cl oder Br, vorzugsweise Cl. Bevorzugte Verbindungen der Formel III sind demzufolge 2,5-Diamino-4,6-dichlor- oder 2,5-Diamino-4,6-dibrompyrimidin.

Geeignet für die Umsetzung sind auch die Salze der 2,5-Diamino-4,6-dihalogenpyrimidine, beispielsweise die Hydrohalogenidsalze wie die Hydrochloridsalze oder die Hydrobromidsalze.

### Beispiel

### Herstellung von 2,5-Diamino-4,6-dichlorpyrimidin

2,5-Diamino-4,6-dihydroxypyrimidinhydrochlorid (0,14 mol, 25 g) wurde in einen trockenen Reaktor gefüllt. Dann wurde trockenes 1,2,3-Trichlorpropan (51,96 ml) hinzugefügt und das Ganze gerührt. Anschliessend wurden Tetramethylammoniumchlorid (0,29 mol, 31,25 g) und dann POCl₃ (0,54 - 0,81 mol, 124,9- 83,28 g, 50,6 - 75,9 ml) hinzugegeben. Die Reaktion wurde auf Rückflusstemperatur (ca. 115 °C) 24 h lang erhitzt. Dann wurde die Reaktion auf unter 50 °C abgekühlt, Eiswasser (24,44 mol, 440,44 g) hinzugefügt und das Ganze unter *55°C* gehalten. Anschliessend wurde die Reaktion auf einen pH-Wert zwischen 6,5 und 7,0 mit 50%iger NaOH (3,12 mol, 124,92 g, 163,3 ml) eingestellt und die Temperatur unter 55 °C gehalten. Die Reaktion wurde 30 min. zwischen 50 und 60 °C gerührt. Dann wurde Tetrahydrofuran (3,7 mol, 267,0 g, 300 ml) zugegeben. Um ungewünschtes Material zu entfernen, wurde das Ganze über Celite abfiltriert und dann der Filterkuchen mit Essigsäureethylester (20,5 mol, 1806,58 g, 2002,86 ml) für die anschliessende Extraktion gewaschen. Die organische Phase (Tetrahydrofuran und Essigsäureethylester) wurde 3mal mit Wasser (5,57 mol, 100,32 g, 100,32 1) gewaschen, über NaHCO₃ getrocknet und filtriert. Essigsäureethylester wurde durch Vakuumdestillation entfernt. Dann wurde Hexan (0,77 mol, 66,14 g, 100,36 ml) zum restlichen organischen Material hinzugegeben, das Ganze auf unter 10 °C abgekühlt, filtriert und dann im Vakuum bei 50 °C getrocknet. Das Titelprodukt (0,09 mol, 15,71 g) wurde als leicht bräunlicher Feststoff erhalten, entsprechend einer Ausbeute von ca. 65 %, bez. auf eingesetztes 2,5-Diamino-4,6-dihydroxypyrimidin.

## Patentansprüche

1. Verfahren zur Herstellung eines 2,5-Diamino-4,6-dihalogenpyrimidins der allgemeinen Formel 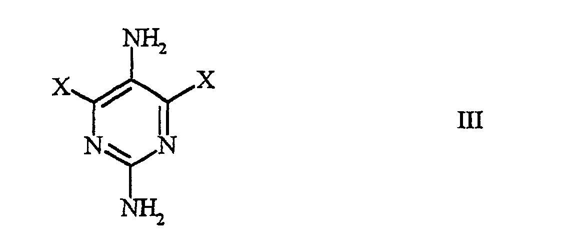 worin X ein Halogenatom bedeutet, welches die Umsetzung von 2,5-Diamino-4,6-dihydroxypyrimidin der Formel 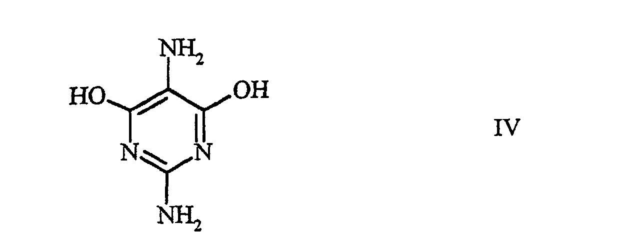 oder einem seiner Salze mit einem Phosphoroxyhalogenid und einem quartären Ammoniumhalogenid umfaßt, **dadurch gekennzeichnet, dass** die Umsetzung in einem halogenierten Alkan als Lösungsmittel durchgeführt wird.

2. Verfahren nach Anspruch 1, **dadurch gekennzeichnet, dass** das halogenierte Alkan ein halogeniertes Propan ist.

3. Verfahren nach Anspruch 2, **dadurch gekennzeichnet, dass** das halogenierte Propan 1,2,3-Trichlorpropan ist.

4. Verfahren nach irgendeinem der Ansprüche 1 bis 3, **dadurch gekennzeichnet, dass** die Umsetzung bei einer Temperatur von 20°C bis zur Rückflußtemperatur des Lösungsmittels durchgeführt wird.

5. Verfahren nach Anspruch 4, **dadurch gekennzeichnet, dass** die Umsetzung bei einer Temperatur von 100 bis 120°C durchgeführt wird.

## Claims

1. Process for preparing a 2,5-diamino-4,6-dihalopyrimidine of the general formula 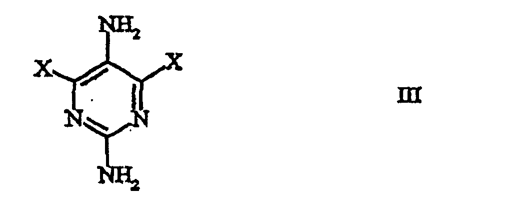 where X is a halogen atom, which comprises the reaction of 2,5-diamino-4,6-dihydroxypyrimidine of the formula 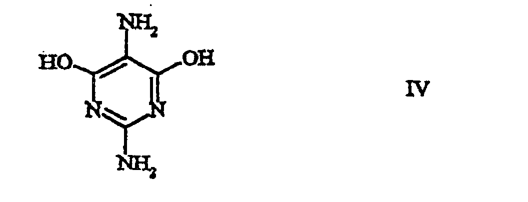 or one of its salts with a phosphorus oxyhalide and a quaternary ammonium halide, **characterized in that** the reaction is carried out in a halogenated alkane as a solvent.

2. Process according to Claim 1, **characterized in that** the halogenated alkane is a halogenated propane.

3. Process according to Claim 2, **characterized in that** the halogenated propane is 1,2,3-trichloropropane.

4. Process according to any of Claims 1 to 3, **characterized in that** the reaction is carried out at a temperature of 20°C up to the reflux temperature of the solvent.

5. Process according to Claim 4, **characterized in that** the reaction is carried out at a temperature of from 100 to 120°C.

## Revendications

1. Procédé de préparation d'une 2,5-diamino-4,6-dihalogénopyrimidine de formule générale 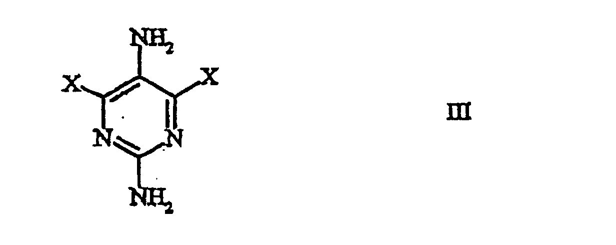 dans laquelle X représente un atome d'halogène, comprenant la réaction de la 2,5-diamino-4,6-dihydroxypyrimidine de formule 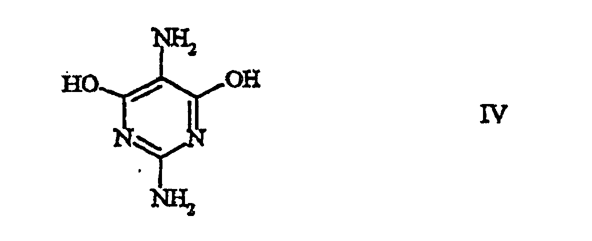 ou de l'un de ses sels avec un oxyhalogénure de phosphore et un halogénure d'ammonium quaternaire, **caractérisé en ce que** la réaction est réalisée dans un alcane halogéné en tant que solvant.

2. Procédé selon la revendication 1, **caractérisé en ce que** l'alcane halogéné est un propane halogéné.

3. Procédé selon la revendication 2, **caractérisé en ce que** le propane halogéné est le 1,2,3-trichloropropane.

4. Procédé selon l'une quelconque des revendications 1 à 3, **caractérisé en ce que** la réaction est réalisée à une température allant de 20°C jusqu'à la température de reflux du solvant.

5. Procédé selon la revendication 4, **caractérisé en ce que** la réaction est réalisée à une température de 100 à 120°C.
